(19) Europäisches Patentamt
European Patent Office
Office européen des brevets

(11) **EP 4 279 829 A1**

(12) **EUROPEAN PATENT APPLICATION**
published in accordance with Art. 153(4) EPC

(43) Date of publication:
**22.11.2023 Bulletin 2023/47**

(21) Application number: **22755828.5**

(22) Date of filing: **21.01.2022**

(51) International Patent Classification (IPC):
**F24F 8/22** *(2021.01)*          **A61L 9/20** *(2006.01)*
**B01D 46/52** *(2006.01)*        **F24F 8/80** *(2021.01)*

(52) Cooperative Patent Classification (CPC):
**A61L 9/20; B01D 46/52; F24F 7/003; F24F 8/22; F24F 8/80**

(86) International application number:
**PCT/JP2022/002169**

(87) International publication number:
**WO 2022/176504 (25.08.2022 Gazette 2022/34)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.02.2021  JP 2021024289**

(71) Applicant: **Daikin Industries, Ltd.**
**Osaka-shi, Osaka 530-0001 (JP)**

(72) Inventors:
• **UMEMURA, Futoshi**
  **Osaka-shi, Osaka 530-8323 (JP)**
• **SEINO, Ryuji**
  **Osaka-shi, Osaka 530-8323 (JP)**
• **NISHIBAYASHI, Kota**
  **Osaka-shi, Osaka 530-8323 (JP)**

(74) Representative: **Hoffmann Eitle**
**Patent- und Rechtsanwälte PartmbB**
**Arabellastraße 30**
**81925 München (DE)**

(54) **AIR CONDITIONER**

(57)    An air conditioner (1) includes a casing (10) that is provided with an air passage (P1) having a suction port (21, 22) and a blow-out port (23), a fan (F) that is disposed in the air passage (P1) and blows out, through the blow-out port (23), air sucked from the suction port (21, 22), an irradiator (70) that is disposed in the casing (10) and emits ultraviolet light, and a filter (31, 32, 33) that is disposed between the blow-out port (23) and the irradiator (70) in the casing (10). The blow-out port (23) of the casing (10) is provided with a blow-out grille (17) having a shielding structure that blocks direct ultraviolet light from the irradiator (70). This attenuates the ultraviolet light leaking out through the blow-out port (23).

*Fig.4*

**Description**

TECHNICAL FIELD

The present disclosure relates to an air conditioner.

BACKGROUND ART

Known as a conventional air conditioner is an air conditioner in which a heat exchanger and a fan are arranged in a casing having a suction port and a blow-out port, and an ultraviolet lamp is disposed upstream of the fan (see, for example, Japanese Unexamined Patent Publication No. H8-312977 (Patent Literature 1)).
In the air conditioner described above, an air flow path that causes the suction port and the blow-out port to communicate with each other is formed in the casing, and a plurality of light shielding plates are arranged upstream of the ultraviolet lamp in the air flow path. The plurality of light shielding plates prevent ultraviolet light from the ultraviolet lamp from leaking out of the casing.

CITATION LIST

PATENT LITERATURE

Patent Literature 1: Japanese Unexamined Patent Publication No. H8-312977

SUMMARY OF INVENTION

TECHNICAL PROBLEMS

The above-described air conditioner, however, requires a space inside the casing where the plurality of light shielding plates are arranged, so that there is a problem that the structure becomes complicated.
The present disclosure proposes an air conditioner that attenuates ultraviolet light leaking out through a blow-out port.

SOLUTIONS TO PROBLEMS

An air conditioner according to the present disclosure includes: a casing that is provided with an air passage having a suction port and a blow-out port; a fan that is disposed in the air passage and blows out, through the blow-out port, air sucked from the suction port; an irradiator that is disposed in the casing and emits ultraviolet light; and a filter that is disposed between the blow-out port and the irradiator in the casing, in which the blow-out port of the casing is provided with a blow-out grille having a shielding structure configured to block direct ultraviolet light from the irradiator.
Here, the "shielding structure configured to block direct ultraviolet light" is a structure configured to attenuate ultraviolet light leaking out of the casing by blocking direct ultraviolet light from the irradiator from directly leaking out of the casing.
According to the present disclosure, the blow-out port of the casing is provided with the blow-out grille having the shielding structure configured to block direct ultraviolet light from the irradiator, so that it is possible to attenuate ultraviolet light leaking out through the blow-out port without providing an extra space in the casing.
Furthermore, in the air conditioner according to an aspect of the present disclosure, the shielding structure of the blow-out grille includes a plurality of plate-shaped members arranged in parallel with each other and spaced apart from each other, and the plurality of plate-shaped members are inclined relative to an optical axis of the irradiator in a cross-sectional view taken along a plane orthogonal to a longitudinal direction of the plate-shaped members.
According to the present disclosure, the plurality of plate-shaped members arranged in parallel with each other and spaced apart from each other are inclined relative to the optical axis of the irradiator in the cross-sectional view taken along the plane orthogonal to the longitudinal direction of the plate-shaped members. This allows the plate-shaped members to block direct ultraviolet light from the irradiator and to attenuate ultraviolet light that has entered a space between the plate-shaped members by causing the ultraviolet light to reflect off the plate-shaped members. It is therefore possible to attenuate ultraviolet light leaking out through the blow-out port with a simple shielding structure.
Furthermore, in the air conditioner according to an aspect of the present disclosure, the plurality of plate-shaped members include a first plate-shaped member group located on one side of the optical axis of the irradiator and a second plate-shaped member group located on the other side of the optical axis in the cross-sectional view, and the first plate-shaped member group and the second plate-shaped member group have different inclination angles.

According to the present disclosure, in the cross-sectional view taken along the plane orthogonal to the longitudinal direction of the plate-shaped members, the first plate-shaped member group and the second plate-shaped member group have different inclination angles, so that it is possible to block direct ultraviolet light from the irradiator with a simple configuration.

Furthermore, in the air conditioner according to an aspect of the present disclosure, the first plate-shaped member group and the second plate-shaped member group are line-symmetric with respect to the optical axis in the cross-sectional view.

According to the present disclosure, in the cross-sectional view taken along the plane orthogonal to the longitudinal direction of the plate-shaped members, the first plate-shaped member group and the second plate-shaped member group are arranged line-symmetrically with respect to the optical axis of the irradiator. Accordingly, the first plate-shaped member group located on one side of the optical axis and the second plate-shaped member group located on the other side of the optical axis are inclined with their respective outer sides closer to the optical axis than their respective inner sides, which allows even a plate-shaped member located away from the optical axis to block direct ultraviolet light from the irradiator.

Furthermore, in the air conditioner according to an aspect of the present disclosure, the plurality of plate-shaped members are configured to cause the plate-shaped members adjacent to each other to partially overlap each other when viewed from a direction orthogonal to an opening surface of the blow-out port.

According to the present disclosure, when viewed from the direction orthogonal to the opening surface of the blow-out port, the plate-shaped members adjacent to each other partially overlap each other, so that direct ultraviolet light from the irradiator is blocked by the plurality of plate-shaped members. It is therefore possible to prevent direct ultraviolet light from leaking out through the blow-out port.

Furthermore, in the air conditioner according to an aspect of the present disclosure, the shielding structure of the blow-out grille is a structure configured to cause the ultraviolet light emitted from the irradiator to reflect twice or more between the plate-shaped members and then go outside.

According to the present disclosure, the ultraviolet light emitted from the irradiator reflects twice or more and then goes outside, so that it is possible to significantly attenuate ultraviolet light leaking out through the blow-out port.

Furthermore, in the air conditioner according to an aspect of the present disclosure, when a thickness of the blow-out grille is denoted by D [mm], a width between the plate-shaped members is denoted by L [mm], and an inclination angle of the plate-shaped members is denoted by θ [deg],

[Math. 1]

$$L \quad < \quad \frac{\cos\theta}{2(\sin\theta)^2} \cdot D$$

is satisfied.

According to the present disclosure, it is possible to easily set, by satisfying the above-described expression, the thickness D of the blow-out grille, the width L between the plate-shaped members, and the inclination angle θ of the plate-shaped members so as to allow the ultraviolet light emitted from the irradiator to reflect twice or more.

Furthermore, in the air conditioner according to an aspect of the present disclosure, illuminance of the ultraviolet light incident on the blow-out grille from the irradiator is less than or equal to $0.2 \times (1/x)^2$ [μW/cm$^2$], where x denotes reflectivity of a material of the blow-out grille.

According to the present disclosure, it is possible to significantly attenuate, by setting the illuminance of the ultraviolet light incident on the blow-out grille from the irradiator less than or equal to $0.2 \times (1/x)^2$ [μW/cm$^2$], ultraviolet light leaking out through the blow-out grille.

Furthermore, in the air conditioner according to an aspect of the present disclosure, the filter is detachably attached to the casing, and the irradiator is configured to stop emitting the ultraviolet light in a state when the filter is removed from the casing.

According to the present disclosure, in a state where the filter is removed from the casing, the irradiator is configured to stop emitting the ultraviolet light, so that, when the filter is removed for maintenance, the ultraviolet light does not leak out of the casing, which improves safety.

BRIEF DESCRIPTION OF DRAWINGS

Fig. 1 is an external perspective view of an air purifier according to an embodiment of the present disclosure.
Fig. 2 is a perspective view of a cross section taken along a line II-II in Fig. 1.
Fig. 3 is an exploded perspective view illustrating a configuration of a main part of the air purifier.
Fig. 4 is a cross-sectional view taken along the line II-II in Fig. 1.
Fig. 5 is a cross-sectional view taken along a line V-V in Fig. 4.
Fig. 6 is a top view of the air purifier.
Fig. 7 is a cross-sectional view of a main part taken along a line VII-VII in Fig. 6.
Fig. 8 is an enlarged cross-sectional view of a main part of a first grille portion of a blow-out grille.
Fig. 9 is a diagram for describing double reflection of ultraviolet light off the blow-out grille.

DESCRIPTION OF EMBODIMENT

Hereinafter, an embodiment will be described. It should be noted that in the drawings, the same reference numerals represent the same or corresponding parts. In addition, the dimensions on the drawings, such as lengths, widths, thicknesses, and depths, are appropriately changed from actual scales for clarity and simplification of the drawings, and do not represent actual relative dimensions. In the drawings, a left-right direction is defined as an X-axis direction, a front-rear direction is defined as a Y-axis direction, and an up-down direction is defined as a Z-axis direction.

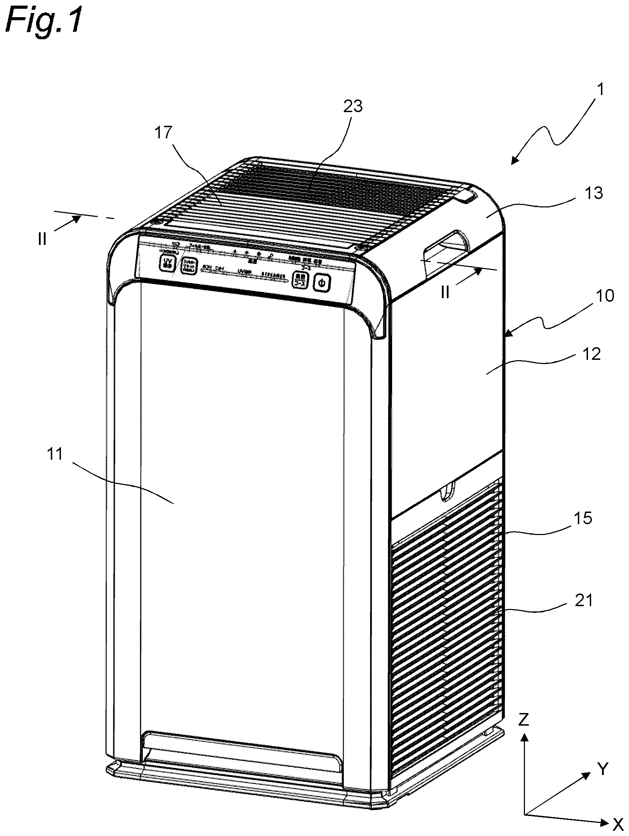

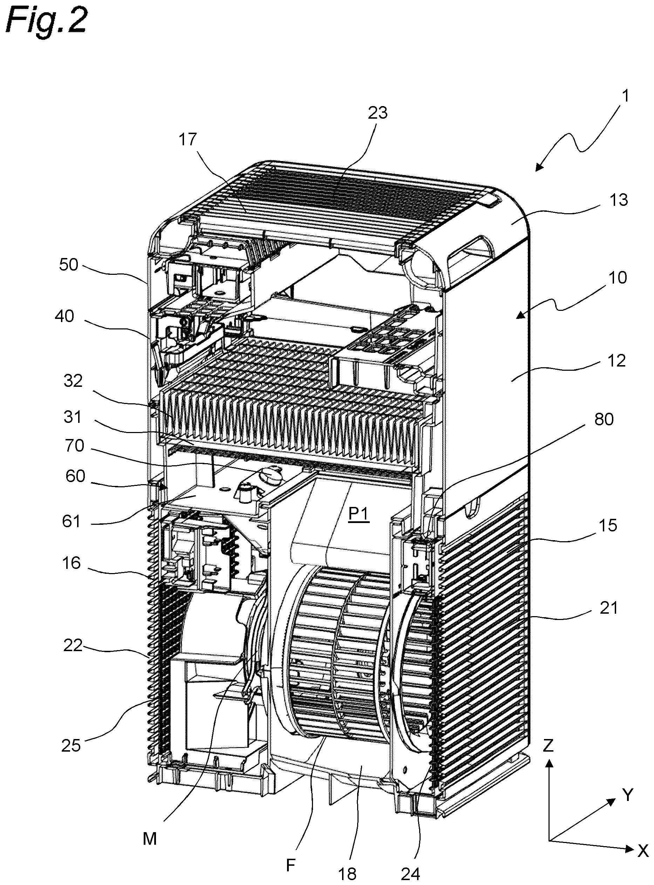

Fig. 1 is an external perspective view of an air purifier 1 according to an embodiment of the present disclosure as viewed from front and obliquely above, and Fig. 2 is a perspective view of a cross section taken along a line II-II in Fig. 1. The air purifier 1 of this embodiment is an example of an air conditioner.

As illustrated in Figs. 1 and 2, the air purifier 1 includes a casing 10 with a rectangular parallelepiped shape, a fan F, a primary filter 31, and a secondary filter 32. The casing 10 is provided with an air passage P1 having a right suction port 21, a left suction port 22, and a blow-out port 23. The fan F is disposed in the air passage P1 and blows out, through the blow-out port 23, air sucked from the right suction port 21 and the left suction port 22. The primary filter 31 is disposed in the air passage P1. The secondary filter 32 is disposed downstream of the primary filter 31 in the air passage P1.

The air purifier 1 further includes an irradiator 70 that irradiates an upstream surface of the primary filter 31 with ultraviolet light, and a streamer unit 80 disposed upstream of the primary filter 31 in the air passage P1.

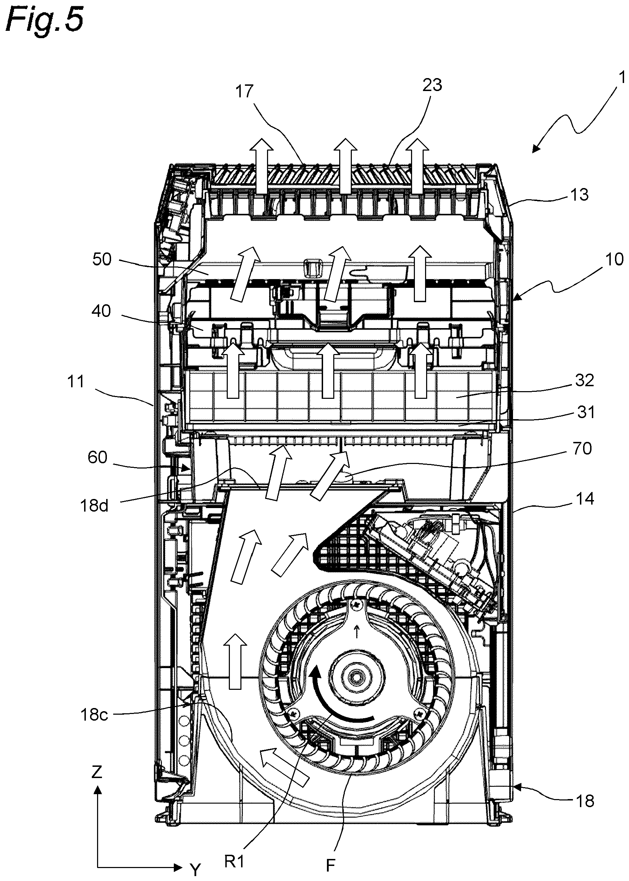

The casing 10 includes a front panel 11, left and right side panels 12, a top panel 13, a rear panel 14 (illustrated in Fig. 5), a right suction grille 15 detachably attached below the right side panel 12, a left suction grille 16 detachably attached below the left side panel 12, and a blow-out grille 17 detachably attached to the top panel 13.

The right suction port 21 is covered with the right suction grille 15 having a grid shape so as to allow air to flow through. The left suction port 22 is covered with the left suction grille 16 having a grid shape so as to allow air to flow through. The blow-out port 23 is covered with blow-out grille 17 having a grid shape so as to allow air to flow through.

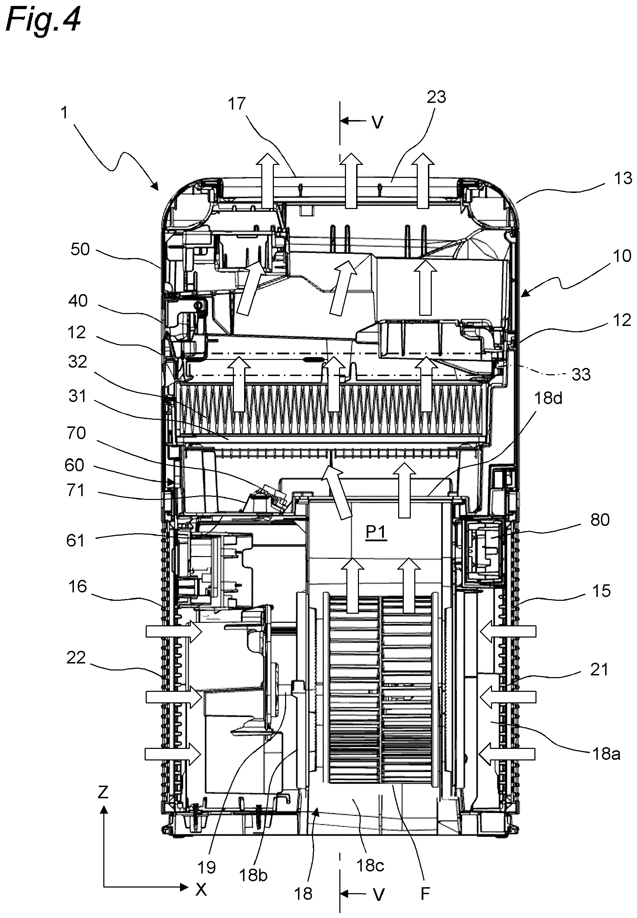

A resin case 40 to which a deodorizing filter 33 (illustrated in Fig. 4) is attached is detachably attached to the casing 10. Furthermore, an upper frame 50 is fitted into the casing 10 above the resin case 40. When the resin case 40 to which the deodorizing filter 33 is attached is removed from the casing 10, the removal of the resin case 40 is detected by a detection device (not illustrated), and a controller (not illustrated) causes the irradiator 70 to stop emitting the ultraviolet light.

Note that the casing 10 is configured to prevent the ultraviolet light from leaking out from the irradiator 70 provided in the casing 10 (in accordance with IEC standard 60335-2-40 and IEC standard 60335-2-65 (illuminance of 0.2 $\mu$W/cm$^2$ or less at a distance of 0.3 m from an outer contour)).

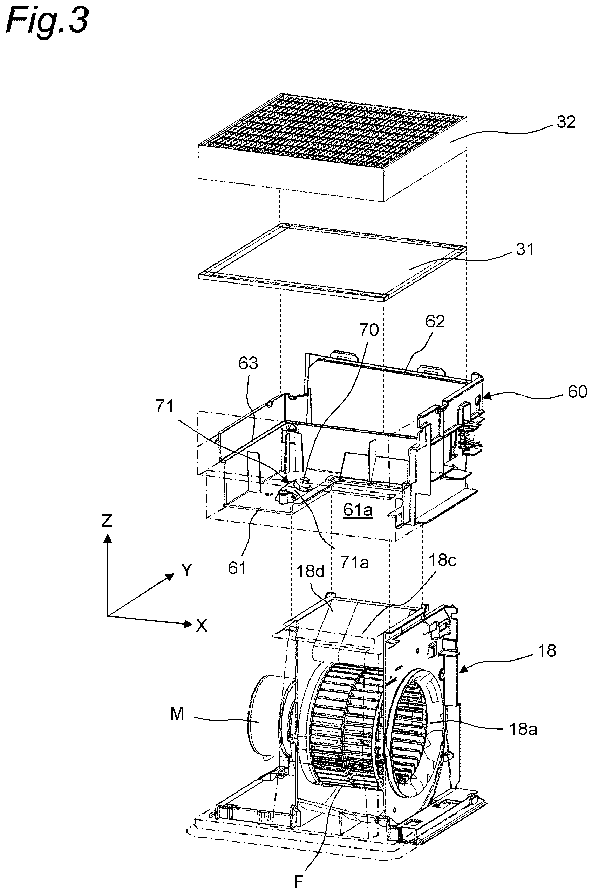

The fan F is a sirocco fan that sucks air from both sides in an axial direction and blows the air radially outward. The fan F is installed in a fan housing 18 having left and right suction ports 18a and 18b (illustrated in Fig. 3 and Fig. 4) and a scroll 18c. The fan housing 18 is provided with a blow-out port 18d through which air sucked from the suction ports 18a and 18b blows upward. A motor M is further provided, the motor M being connected to a left side of the fan F via a shaft 19 (illustrated in Fig. 4).

In the casing 10, a lower frame 60 is disposed above the fan housing 18, the lower frame 60 having a box shape and being opened upward. The lower frame 60 and the upper frame 50 form a space in which the resin case 40 is installed.

The irradiator 70 that irradiates the upstream surface of the primary filter 31 with the ultraviolet light is attached to a bottom portion 61 of the lower frame 60.

The primary filter 31 is a filter capable of removing particles having a particle diameter of 10 $\mu$m to 50 $\mu$m. A thickness of the primary filter 31 is set so as to allow the ultraviolet light from the irradiator 70 to reach a downstream surface of the primary filter 31 (for example, a thickness of about 5 mm to 10 mm). Here, the irradiator 70 includes a light emitting diode (LED) that emits deep ultraviolet light UV-C in a wavelength range of 100 nm to 280 nm.

Note that, in this embodiment, the irradiator 70 that emits the deep ultraviolet light UV-C in the wavelength range of 100 nm to 280 nm is used, but any irradiator that emits ultraviolet light within a wavelength range of 100 nm to 400 nm may be used. Alternatively, an ultraviolet lamp or the like may be used as the irradiator.

The secondary filter 32 is a filter that has a pleated structure and traps particles having a particle diameter of 0.7 $\mu$m. For example, the secondary filter 32 may be a high efficiency particulate air (HEPA) filter that traps 99.97% or more of particles having a particle diameter of 0.3 $\mu$m or a medium efficiency particulate air filter that traps particles having a particle diameter of 0.4 $\mu$m to 0.7 $\mu$m. The secondary filter 32 is impregnated with a chemical agent exhibiting antiviral properties. For example, a lytic enzyme that destroys the envelopes of viruses to inactivate the viruses is used as the chemical agent.

Note that the secondary filter may be impregnated with a chemical agent exhibiting antibacterial properties to inhibit the growth of bacteria, or both the chemical agent exhibiting antiviral properties and the chemical agent exhibiting antibacterial properties may be used. Examples of the chemical agent exhibiting antiviral properties and antibacterial properties include Ag, an enzyme, ammonia, and the like, and a chemical agent containing a mixture of at least two of Ag, an enzyme, ammonia, and the like may be used.

Net-like pre-filters 24 and 25 for removing relatively large dust are attached to a leeward surface of the right suction grille 15 and a leeward surface of the left suction grille 16, respectively. The pre-filters 24 and 25 are each disposed upstream of the primary filter 31 in the air passage P1. The pre-filters 24 and 25 trap dust larger than particles trapped by the primary filter 31.

The air passage P1 is formed in the casing 10. Air sucked in the air passage P1 from the right suction port 21 and the left suction port 22 blows out from the blow-out port 23 via the fan F, the primary filter 31, and the secondary filter 32.

Fig. 3 is an exploded perspective view illustrating a configuration of a main part of the air purifier 1. Note that, in Fig. 3, the lower frame 60 and the fan housing 18 are each illustrated as a cross section taken along the line II-II in Fig. 1.

As illustrated in Fig. 3, the lower frame 60 includes the bottom portion 61 having an opening 61a to which the blow-out port 18d of the fan housing 18 is connected, and a wall portion 62 extending upward from an outer peripheral edge of the bottom portion 61. In the wall portion 62, a peripheral step 63 is formed at a distance from the bottom portion 61. The primary filter 31 and the secondary filter 32 are supported by an upper surface of the peripheral step 63 with the secondary filter 32 stacked on top of the primary filter 31.

A base 71 having an inclined surface 71a is provided on a surface of the bottom portion 61 of the lower frame 60 facing the primary filter 31. The irradiator 70 is attached to the inclined surface 71a of the base 71.

Fig. 4 is a cross-sectional view taken along the line II-II in Fig. 1. In Fig. 4, arrows indicate the flow of air through the air passage P1.

As illustrated in Fig. 4, when the fan F is driven by the motor M, air sucked by the fan F from the right suction port 21 and the left suction port 22 blows upward from the fan F and then blows upward from the blow-out port 23 through the primary filter 31, the secondary filter 32, and the deodorizing filter 33.

Fig. 5 is a cross-sectional view taken along a line V-V in Fig. 4, and in Fig. 5, when the fan F rotates in a clockwise direction (arrow R1), air sucked from both sides in a direction orthogonal to the page blows radially outward of the fan F and is straightened by the scroll 18c of the fan housing 18 to blow upward from the blow-out port 18d.

<Shielding structure of blow-out grille 17>

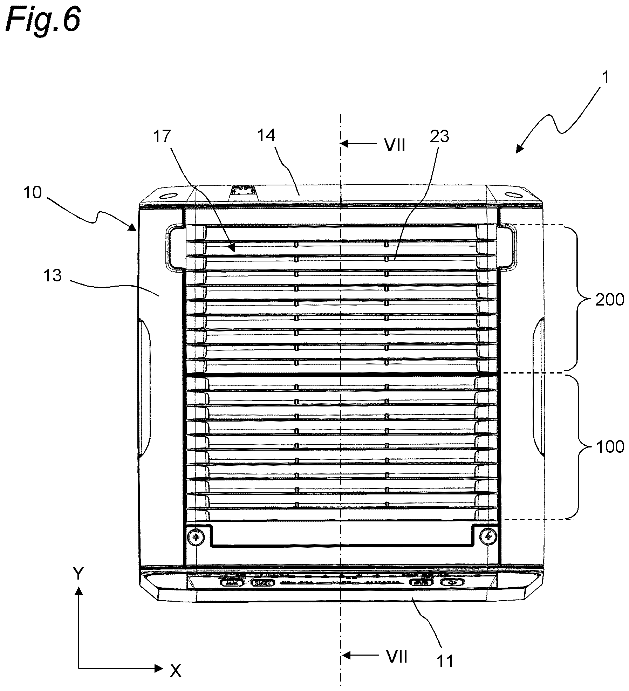

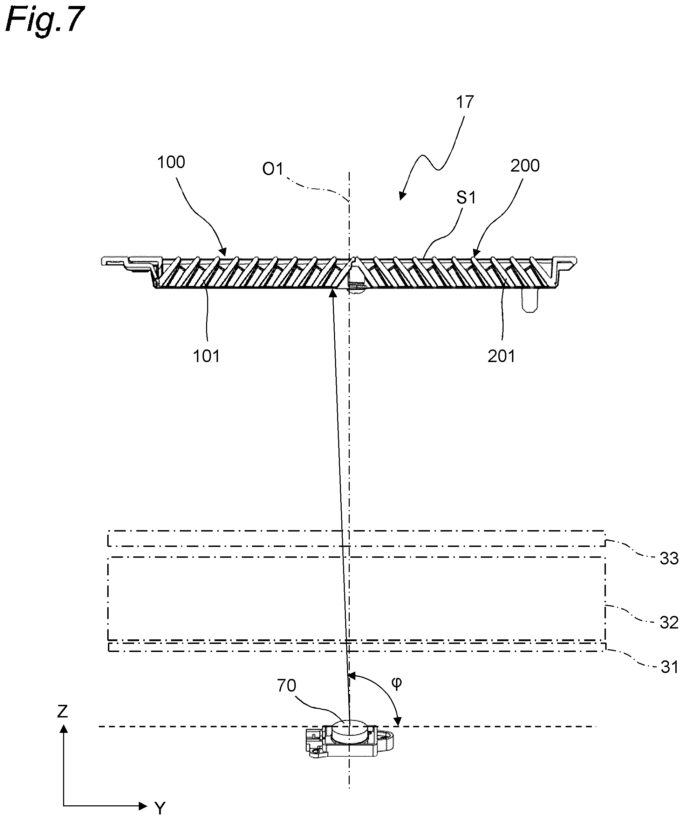

Fig. 6 is a top view of the air purifier 1, and Fig. 7 is a cross-sectional view of a main part taken along a line VII-VII in Fig. 6. Fig. 7 is a cross-sectional view taken along a plane orthogonal to a longitudinal direction of a plate-shaped member 101.

As illustrated in Figs. 6 and 7, the blow-out grille 17 includes a first grille portion 100 that covers a front half (lower side of Fig. 6) of the rectangular blow-out port 23, and a second grille portion 200 that covers a rear half (upper side of Fig. 6) of the blow-out port 23. The first grille portion 100 and the second grille portion 200 form a shielding structure that blocks direct ultraviolet light. The first grille portion 100 includes a plurality of plate-shaped members 101 (illustrated in Fig. 7) as an example of a first plate-shaped member group. The second grille portion 200 includes a plurality of plate-shaped members 201 (illustrated in Fig. 7) as an example of a second plate-shaped member group.

The plurality of plate-shaped members 101 are configured to cause the plate-shaped members 101 adjacent to each other to partially overlap each other when viewed from a direction orthogonal to an opening surface S1 (illustrated in Fig. 7) of the blow-out port 23. Furthermore, the plurality of plate-shaped members 201 are configured to cause the plate-shaped members 201 adjacent to each other to partially overlap each other when viewed from the direction orthogonal to the opening surface S1 of the blow-out port 23.

Note that, in this embodiment, the opening surface S1 of the blow-out port 23 is a flat surface, or alternatively, the opening surface of the blow-out port may be a curved surface or the like. In this case, it is sufficient that the plate-shaped members adjacent to each other partially overlap each other when viewed from a direction orthogonal to a tangential plane tangent to the curved surface.

As illustrated in Fig. 7, the first grille portion 100 has the plurality of plate-shaped members 101 arranged in parallel with each other and spaced apart from each other in the front-rear direction (Y-axis direction). A longitudinal direction of each plate-shaped member 101 coincides with a direction (X-axis direction) orthogonal to the page. Each plate-shaped member 101 is inclined such that a direction from a lower edge to an upper edge extends rearward and obliquely upward. In other words, each plate-shaped member 101 is inclined relative to the opening surface S 1 with an upper end portion positioned at a rear side relative to a lower end portion.

Furthermore, the second grille portion 200 has the plurality of plate-shaped members 201 arranged in parallel with each other and spaced apart from each other in the front-rear direction (Y-axis direction). A longitudinal direction of each plate-shaped member 201 coincides with the left-right direction (X-axis direction). Each plate-shaped member 201 is inclined such that a direction from a lower edge to an upper edge extends frontward and obliquely upward. In other words, each plate-shaped member 201 is inclined relative to the opening surface S1 with an upper end portion positioned at a front side relative to a lower end portion.

Each plate-shaped member 101 of the first grille portion 100 is inclined relative to an optical axis O1 of the irradiator 70 in a cross-sectional view taken along a plane orthogonal to the longitudinal direction of the plate-shaped members 101. In this embodiment, a space between plate-shaped members 101 closest to the optical axis O1 has the smallest incident angle $\varphi$ (in this embodiment, $\varphi$ = 93 degrees).

The plurality of plate-shaped members 101 of the first grille portion 100 and the plurality of plate-shaped members 201 of the second grille portion 200 are arranged line-symmetrically with respect to the optical axis O1 of the irradiator 70 in the cross-sectional view taken along the plane orthogonal to the longitudinal direction of the plate-shaped members 101.

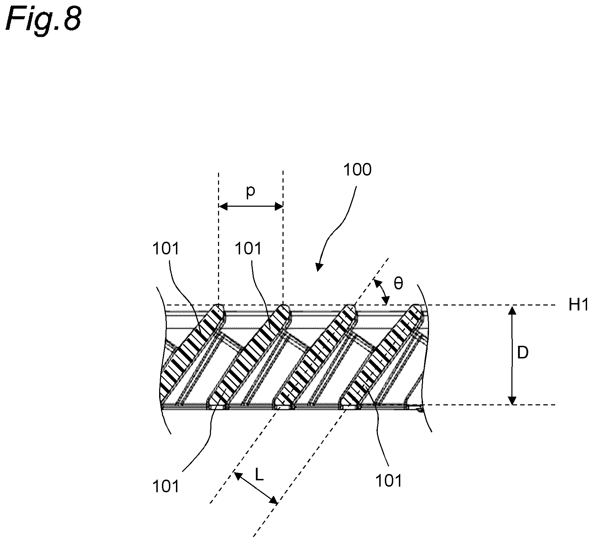

Fig 8 is an enlarged cross-sectional view of a main part of the first grille portion 100 of the blow-out grille 17. In Fig. 8, a pitch between the plate-shaped members 101 of the first grille portion 100 is denoted by p, a thickness of the first grille portion 100 is denoted by D, a width between the plate-shaped members 101 is denoted by L, and an inclination angle of the plate-shaped members 101 relative to a plane H1 parallel to the X axis and the Y axis is denoted by $\theta$.

Here, with the thickness D, the width L, and the inclination angle $\theta$ determined to satisfy the following condition:
[Math. 2]

$$L < \frac{\cos\theta}{2(\sin\theta)^2} \cdot D \qquad \cdots\cdots (1)$$
,

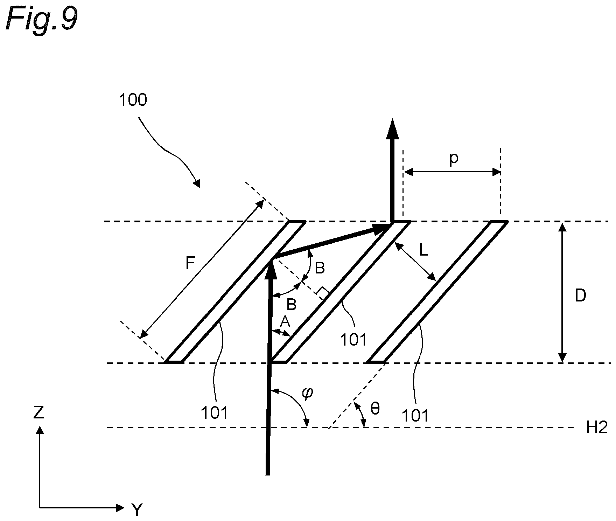

when the incident angle $\varphi$ of the ultraviolet light is 90 degrees as illustrated in Fig. 9, the ultraviolet light reflects at least twice. The incident angle $\varphi$ is an angle formed by a plane H2 parallel to the X axis and the Y axis and the ultraviolet light that enters a space between the plate-shaped members 101 in the cross-sectional view taken along the plane orthogonal to the longitudinal direction of the plate-shaped members 101.

With reference to Figs. 8 and 9, the plate-shaped members 101 of the first grille portion have been described, but the same applies to the plate-shaped members 201 of the second grille portion 200 arranged line-symmetrically with respect to the optical axis O1 of the irradiator 70.

Hereinafter, how to derive the above-described expression (1) will be described.

First, an angle A and an angle B illustrated in Fig. 9 are expressed as follows:

[Math. 3]

$$A = \varphi - \theta$$
$$B = 90 - A = |90 - \varphi + \theta|$$.

Further, a length F of each plate-shaped member 101 is expressed as follows:
[Math. 4]

$$F = 2L\tan B = 2L\tan|90 - \varphi + \theta| = \left|\frac{2L}{\tan(\varphi - \theta)}\right| \qquad \cdots\cdots (2)$$.

the thickness D of the first grille portion 100 is expressed as follows:

[Math. 5]

$$D = F \sin \theta ,$$

which is transformed into:

[Math. 6]

$$F = \frac{D}{\sin \theta} .$$

When this is substituted into the above-described expression (2),

[Math. 7]

$$\frac{D}{\sin \theta} = \left| \frac{2L}{\tan(\varphi - \theta)} \right|$$

$$D = \left| \frac{2L \sin \theta}{\tan(\varphi - \theta)} \right|$$

are given, and the width L between the plate-shaped members 101 is expressed as follows:
[Math. 8]

$$L = \left| \frac{\tan(\varphi - \theta)}{2 \sin \theta} \right| \quad \cdots \cdots (3)$$

Here, when that the incident angle $\varphi$ of the ultraviolet light is set at 90 degrees that is the strictest condition under which the ultraviolet light reflects twice,

[Math. 9]

$$\tan(\varphi - \theta) = \frac{1}{\tan \theta} = \frac{\cos \theta}{\sin \theta}$$

is given, and this expression is substituted into the above-described expression (3) to derive the above-described expression (1).

In the air purifier 1 having the above-described configuration, the blow-out port 23 of the casing 10 is provided with the blow-out grille 17 having the shielding structure that blocks direct ultraviolet light from the irradiator 70, so that it is possible to attenuate direct ultraviolet light leaking out through the blow-out port 23 without providing an extra space in the casing 10.

Furthermore, as illustrated in Fig. 7, the plurality of plate-shaped members 101 and 201 arranged in parallel with each other and spaced apart from each other are inclined relative to the optical axis O1 of the irradiator 70 in the cross-sectional view taken along the plane orthogonal to the longitudinal direction of the plate-shaped members 101 and 201 to block direct ultraviolet light from the irradiator 70 and to attenuate ultraviolet light that has entered a space between the plate-shaped members 101 and 201 by causing the ultraviolet light to reflect off the plate-shaped members 101 and 201. This makes it possible to attenuate ultraviolet light leaking out through the blow-out port 23 with a simple shielding structure.

Furthermore, in the cross-sectional view taken along the plane orthogonal to the longitudinal direction of the plate-shaped members 101 and 201, the plurality of plate-shaped members 101 (first plate-shaped member group) of the first grille portion 100 located on one side of the optical axis O1 of the irradiator 70 and the plurality of plate-shaped members 201 (second plate-shaped member group) of the second grille portion 200 located on the other side of the optical axis O1 have different inclination angles each other, so that it is possible to block direct ultraviolet light from the irradiator 70 with a simple configuration.

Furthermore, in the cross-sectional view taken along the plane orthogonal to the longitudinal direction of the plate-shaped members 101 and 201, the plurality of plate-shaped members 101 of the first grille portion 100 and the plurality of plate-shaped members 201 of the second grille portion 200 are arranged line-symmetrically with respect to the optical axis O1 of the irradiator 70. Accordingly, the plurality of plate-shaped members 101 located on one side of the optical axis O1 and the plurality of plate-shaped members 201 located on the other side of the optical axis O1 are inclined with their respective upper sides closer to the optical axis O1, which allows even a plate-shaped member located away from the optical axis O1 to block direct ultraviolet light from the irradiator 70.

Furthermore, when viewed from the direction orthogonal to the opening surface S1 of the blow-out port 23, the plate-shaped members 101 adjacent to each other partially overlap each other, and the plate-shaped members 201 adjacent to each other partially overlap each other, so that direct ultraviolet light from the irradiator 70 is blocked by the plurality of plate-shaped members 101 and 201, and it is therefore possible to prevent direct ultraviolet light from leaking out through the blow-out port 23.

Furthermore, the shielding structure of the blow-out grille 17 causes the ultraviolet light emitted from the irradiator 70 to reflect twice or more and then go outside, so that it is possible to significantly attenuate ultraviolet light leaking out through the blow-out port 23.

Furthermore, it is possible to easily set, by satisfying the above-described expression (1), the thickness D of the blow-out grille 17, the width L between the plate-shaped members 101 and 201, and the inclination angle $\theta$ of the plate-shaped members 101 and 201 so as to allow the ultraviolet light emitted from the irradiator 70 to reflect twice or more.

Furthermore, it is possible to significantly attenuate, by setting the illuminance of the ultraviolet light incident on the blow-out grille 17 from the irradiator 70 less than or equal to $0.2 \times (1/x)^2$ [$\mu$W/cm$^2$], ultraviolet light leaking out through the blow-out grille 17. Furthermore, the illuminance of the ultraviolet light leaking out through the blow-out port 23 can satisfy the IEC standard (illuminance of 0.2 [$\mu$W/cm$^2$] or less at a distance of 0.3 m from an outer contour). Note that the illuminance of the ultraviolet light incident on the blow-out grille 17 is illuminance of ultraviolet light at the lower end portions of the plate-shaped members 101 and 201 of the blow-out grille 17. Furthermore, the x denotes reflectivity of a material of the blow-out grille 17 against ultraviolet light and is a predetermined value that varies in a manner that depends on the material of the blow-out grille.

Furthermore, in a state where the deodorizing filter 33 is removed together with the resin case 40 from the casing 10, the irradiator 70 stops emitting the ultraviolet light, so that, when the deodorizing filter 33 is removed for maintenance, the ultraviolet light does not leak out of the casing 10, which improves safety.

Note that the primary filter 31 and the secondary filter 32 are taken out from an opening of the casing 10 that is opened when the resin case 40 is removed.

In the above-described embodiment, the air purifier 1 has been described as an example of the air conditioner, but the present disclosure may be applied to an air conditioner having a cooling function or a heating function.

In the above-described embodiment, the air purifier 1 in which the fan F is disposed upstream of the primary filter 31, the secondary filter 32, and the deodorizing filter 33 has been described, but the fan may be disposed downstream of the primary filter 31, the secondary filter 32, and the deodorizing filter 33.

Although a specific embodiment of the present disclosure has been described, the present disclosure is not limited to the above-described embodiment, and various modifications can be made within the scope of the present disclosure.

REFERENCE SIGNS LIST

| 1 | air purifier |
| 10 | casing |
| 11 | front panel |
| 12 | side panel |
| 13 | top panel |
| 14 | rear panel |
| 15 | right suction grille |
| 16 | left suction grille |
| 17 | blow-out grille |

| | |
|---|---|
| 18 | fan housing |
| 18a, 18b | suction port |
| 18c | scroll |
| 18d | blow-out port |
| 19 | shaft |
| 21 | right suction port |
| 22 | left suction port |
| 23 | blow-out port |
| 24, 25 | pre-filter |
| 31 | primary filter |
| 32 | secondary filter |
| 33 | deodorizing filter |
| 40 | resin case |
| 50 | upper frame |
| 60 | lower frame |
| 61 | bottom portion |
| 61a | opening |
| 62 | wall portion |
| 63 | peripheral step |
| 70 | irradiation unit |
| 71 | base |
| 71a | inclined surface |
| 80 | streamer unit |
| 100 | first grille portion |
| 101 | plurality of plate-shaped members (first plate-shaped member group) |
| 200 | second grille portion |
| 201 | plurality of plate-shaped members (second plate-shaped member group) |
| F | fan |
| M | motor |
| O1 | optical axis |
| P1 | air passage |

**Claims**

1. An air conditioner comprising:

   a casing (10) that is provided with an air passage (P1) having a suction port (21, 22) and a blow-out port (23);
   a fan (F) that is disposed in the air passage (P1) and blows out, through the blow-out port (23), air sucked from the suction port (21, 22);
   an irradiator (70) that is disposed in the casing (10) and emits ultraviolet light; and
   a filter (31, 32, 33) that is disposed between the blow-out port (23) and the irradiator (70) in the casing (10),
   wherein the blow-out port (23) of the casing (10) is provided with a blow-out grille (17) having a shielding structure configured to block direct ultraviolet light from the irradiator (70).

2. The air conditioner according to claim 1, wherein

   the shielding structure of the blow-out grille (17) includes a plurality of plate-shaped members (101, 201) arranged in parallel with each other and spaced apart from each other, and
   the plurality of plate-shaped members (101, 201) are inclined relative to an optical axis (O1) of the irradiator (70) in a cross-sectional view taken along a plane orthogonal to a longitudinal direction of the plate-shaped members (101, 201).

3. The air conditioner according to claim 2, wherein

   the plurality of plate-shaped members (101, 201) include a first plate-shaped member group (101) located on one side of the optical axis (O1) of the irradiator (70) and a second plate-shaped member group (201) located on another side of the optical axis (O1) in the cross-sectional view, and

inclination angles of the first plate-shaped member group (101) are different from inclination angles of the second plate-shaped member group (201).

4. The air conditioner according to claim 3, wherein the first plate-shaped member group (101) and the second plate-shaped member group (201) are line-symmetric with respect to the optical axis (O1) in the cross-sectional view.

5. The air conditioner according to any one of claims 2 to 4, wherein the plurality of plate-shaped members (101, 201) are configured to cause the plate-shaped members (101, 201) adjacent to each other to partially overlap each other when viewed from a direction orthogonal to an opening surface (S 1) of the blow-out port (23).

6. The air conditioner according to any one of claims 1 to 5, wherein the shielding structure of the blow-out grille (17) is a structure configured to cause the ultraviolet light emitted from the irradiator (70) to reflect twice or more and then go outside.

7. The air conditioner according to any one of claims 2 to 4, wherein
when a thickness of the blow-out grille (17) is denoted by D [mm], a width between the plate-shaped members (101, 201) is denoted by L [mm], and an inclination angle of the plate-shaped members (101, 201) is denoted by θ [deg],

[Math. 1]

$$ L \ < \ \frac{\cos\theta}{2(\sin\theta)^2} \cdot D $$

is satisfied.

8. The air conditioner according to claim 6 or 7, wherein illuminance of the ultraviolet light incident on the blow-out grille (17) from the irradiator (70) is less than or equal to $0.2 \times (1/x)^2$ [μW/cm$^2$], where x denotes reflectivity of a material of the blow-out grille (17).

9. The air conditioner according to any one of claims 1 to 8, wherein

the filter (33) is detachably attached to the casing (10), and
the irradiator (70) is configured to stop emitting the ultraviolet light in a state where the filter (33) is removed from the casing (10).

*Fig.1*

*Fig.2*

*Fig.3*

# Fig.4

*Fig.5*

*Fig.6*

*Fig.7*

*Fig.8*

Fig.9

## INTERNATIONAL SEARCH REPORT

| International application No. |
|---|
| **PCT/JP2022/002169** |

**A. CLASSIFICATION OF SUBJECT MATTER**

*F24F 8/22*(2021.01)i; *A61L 9/20*(2006.01)i; *B01D 46/52*(2006.01)i; *F24F 8/80*(2021.01)i
FI: F24F8/22; A61L9/20; B01D46/52 Z; F24F8/80 238

According to International Patent Classification (IPC) or to both national classification and IPC

**B. FIELDS SEARCHED**

Minimum documentation searched (classification system followed by classification symbols)

F24F8/22; A61L9/20; B01D46/52; F24F8/80

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2007-500055 A (ECO RX INC.) 11 January 2007 (2007-01-11)<br>    paragraphs [0020]-[0077], fig. 1-10 | 1 |
| Y | | 2-9 |
| Y | Microfilm of the specification and drawings annexed to the request of Japanese Utility Model Application No. 18022/1980 (Laid-open No. 119940/1981) (SHINKO KOGYO CO., LTD.) 12 September 1981 (1981-09-12), fig. 5 (a)-6 (c) | 2-9 |
| Y | JP 2002-45415 A (DENSO CORP.) 12 February 2002 (2002-02-12)<br>    paragraphs [0036]-[0045], fig. 5, 6 | 5-9 |
| Y | JP 7-299294 A (MITSUBISHI ELECTRIC HOME APPLIANCE CO., LTD.) 14 November 1995 (1995-11-14)<br>    paragraphs [0032]-[0034], fig. 2, 3 | 9 |
| A | JP 8-720 A (MATSUSHITA ELECTRIC WORKS LTD.) 09 January 1996 (1996-01-09)<br>    entire text, all drawings | 1-9 |
| A | JP 11-104225 A (SHARP KK) 20 April 1999 (1999-04-20)<br>    entire text, all drawings | 1-9 |

☐ Further documents are listed in the continuation of Box C.　　☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **01 March 2022** | **15 March 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2022/002169**

| Patent document cited in search report | | | Publication date (day/month/year) | Patent family member(s) | Publication date (day/month/year) |
|---|---|---|---|---|---|
| JP | 2007-500055 | A | 11 January 2007 | US 2005/0000365 A1 paragraphs [0033]-[0090], fig. 1-10 WO 2004/101101 A2 KR 10-2006-0026404 A CN 1805775 A | |
| JP | 56-119940 | U1 | 12 September 1981 | (Family: none) | |
| JP | 2002-45415 | A | 12 February 2002 | (Family: none) | |
| JP | 7-299294 | A | 14 November 1995 | (Family: none) | |
| JP | 8-720 | A | 09 January 1996 | (Family: none) | |
| JP | 11-104225 | A | 20 April 1999 | (Family: none) | |

Form PCT/ISA/210 (patent family annex) (January 2015)

**EP 4 279 829 A1**

**REFERENCES CITED IN THE DESCRIPTION**

**Patent documents cited in the description**

• JP H8312977 A